# EUROPEAN PATENT APPLICATION

(11) **EP 4 751 670 A1**
(43) Date of publication of application: **03.06.2026**
(21) Application number: 25215777.1
(22) Date of filing: 14.11.2025
(51) Int. Cl.: A61B 18/14

(54) **FLEX-CIRCUIT ARRANGEMENT FOR THERMOCOUPLES IN CATHETER**

(30) Priority: 27.11.2024 US 202418961531
(71) Applicant: Biosense Webster (Israel) Ltd., 2066717 Yokneam (IL)
(72) Inventor: SUAREZ, Paul A., Irvine, 92618 (US); RORICK, Kevin, Irvine, 92618 (US); PARSA, Sean, Irvine, 92618 (US); PHAM, Cuong Gia, Irvine, 92618 (US); THALER, Sean D., Irvine, 92618 (US)
(74) Representative: Carpmaels & Ransford LLP

(57) **Abstract**

A catheter includes a shaft assembly, an end effector, and a flexible circuit. The end effector is positioned at a distal end of the shaft assembly and includes a tip member. The tip member is operable to apply electrical energy to tissue. The tip member defines a bore, which has an inner sidewall. The flexible circuit includes a thermocouple positioned within the bore of the tip member. The thermocouple is configured to sense a temperature of the tip member. The flexible circuit further includes a deformable feature that resiliently urges the thermocouple toward the inner sidewall of the bore.

## Description

### BACKGROUND

Cardiac arrhythmias, such as atrial fibrillation, occur when regions of cardiac tissue abnormally conduct electric signals. Procedures for treating arrhythmia include surgically disrupting the conducting pathway for such signals. By selectively ablating cardiac tissue by application of energy (e.g., alternating-current or direct-current energy), it may be possible to cease or modify the propagation of unwanted electrical signals from one portion of the heart to another. The ablation process may provide a barrier to unwanted electrical pathways by creating electrically insulative lesions or scar tissue that effectively block communication of aberrant electrical signals across the tissue.

In some procedures, a catheter with one or more electrodes may be used to provide ablation within the cardiovascular system. The catheter may be inserted into a major vein or artery (e.g., the femoral artery) and then advanced to position the electrodes within the heart or in a cardiovascular structure adjacent to the heart (e.g., the pulmonary vein). The one or more electrodes may be placed in contact with cardiac tissue or other vascular tissue and then activated with electrical energy to thereby ablate the contacted tissue. In some cases, the electrodes may be bipolar. In some other cases, a monopolar electrode may be used in conjunction with a ground pad or other reference electrode that is in contact with the patient. Irrigation may be used to draw heat from ablating components of an ablation catheter; and to prevent the formation of blood clots near the ablation site.

Examples of ablation catheters are described in U.S. Pat. No. 10,743,932, entitled "Integrated Ablation System using Catheter with Multiple Irrigation Lumens," issued August 18, 2020, the disclosure of which is incorporated by reference herein, in its entirety; U.S. Pat. No. 10,660,700, entitled "Irrigated Balloon Catheter with Flexible Circuit Electrode Assembly," issued May 26, 2020, the disclosure of which is incorporated by reference herein, in its entirety; U.S. Pat. No. 11,559,349, entitled "Ablation Catheter with a Flexible Printed Circuit Board," issued January 24, 2023, the disclosure of which is incorporated by reference herein, in its entirety; U.S. Pat. No. 10,702,177, entitled "Catheter with Bipole Electrode Spacer and Related Methods," issued July 7, 2020, the disclosure of which is incorporated by reference herein, in its entirety; U.S. Pat. No. 10,130,422, entitled "Catheter with Soft Distal Tip for Mapping and Ablating Tubular Region," issued November 20, 2018, the disclosure of which is incorporated by reference herein, in its entirety; U.S. Pat. No. 8,956,353, entitled "Electrode Irrigation Using Micro-Jets," issued February 17, 2015, the disclosure of which is incorporated by reference herein, in its entirety; and U.S. Pat. No. 9,801,585, entitled "Electrocardiogram Noise Reduction," issued October 31, 2017, the disclosure of which is incorporated by reference herein, in its entirety.

Some catheter ablation procedures may be performed after using electrophysiology (EP) mapping to identify tissue regions that should be targeted for ablation. Such EP mapping may include the use of sensing electrodes on a catheter (e.g., the same catheter that is used to perform the ablation or a dedicated mapping catheter). Such sensing electrodes may monitor electrical signals emanating from conductive endocardial tissues to pinpoint the location of aberrant conductive tissue sites that are responsible for the arrhythmia. Examples of an EP mapping system are described in U.S. Pat. No. 5,738,096, entitled "Cardiac Electromechanics," issued April 14, 1998, the disclosure of which is incorporated by reference herein, in its entirety. Examples of EP mapping catheters are described in U.S. Pat. No. 9,907,480, entitled "Catheter Spine Assembly with Closely-Spaced Bipole Microelectrodes," issued March 6, 2018, the disclosure of which is incorporated by reference herein, in its entirety; U.S. Pat. No. 10,130,422, entitled "Catheter with Soft Distal Tip for Mapping and Ablating Tubular Region," issued November 20, 2018, the disclosure of which is incorporated by reference herein, in its entirety; and U.S. Pat. No. 10,702,177, entitled "Catheter with Bipole Electrode Spacer and Related Methods," issued July 7, 2020, the disclosure of which is incorporated by reference herein, in its entirety.

When using an ablation catheter, it may be desirable to monitor the temperature at one or more distal tip regions of the catheter, which may further indicate the temperature of tissue in contact with the distal tip of the catheter. Temperature monitoring may be conducted through one or more thermocouples integrated into the catheter. Each thermocouple may be capable of indicating a measured temperature to a processor. In some cases, thermocouples for catheters may be difficult to manufacture and to position for accurate temperature readings because of their small size. This difficulty may tend to provide undesirably high scrap rates during manufacturing.

While several catheter systems and methods have been made and used, it is believed that no one prior to the inventors has made or used the invention described, illustrated and claimed herein.

### BRIEF DESCRIPTION OF THE DRAWINGS

The drawings and detailed description that follow are intended to be merely illustrative and are not intended to limit the scope of the invention as contemplated by the inventors.
FIG. 1 depicts a schematic view of a medical procedure in which a catheter of a catheter assembly is inserted in a patient;
FIG. 2 depicts a perspective view of a distal portion of the catheter of FIG. 1, with additional components shown in schematic form;
FIG. 3 depicts an exploded perspective view of a tip member and flexible circuit of the catheter of FIG. 1;
FIG. 3A depicts a perspective view of the tip member of FIG. 3 with the flexible circuit of FIG. 3 inserted, with a portion of the tip member in cross-section;
FIG. 4 depicts a cross-section side view of the tip member and flexible circuit of FIG. 3, taken along line 4-4 of FIG. 3A;
FIG. 5 depicts an exploded perspective view of the tip member, flexible circuit, and a printed circuit board of the catheter of FIG. 1;
FIG. 6 depicts a cross-section side view of a distal portion of the flexible circuit of FIG. 3;
FIG. 7 depicts a cross-section end view of the tip member and flexible circuit of FIG. 3, taken along line 7-7 of FIG. 3A;
FIG. 8 depicts an exploded perspective view of the tip member of FIG. 3 and a second flexible circuit, with a distal portion of fourth flexible circuit in a pre-folded configuration;
FIG. 8A depicts a perspective view of the tip member of FIG. 3 with the second flexible circuit of FIG. 8 inserted, and with a portion of the tip member in cross-section;
FIG. 9 depicts a cross-section side view of the tip member and second flexible circuit of FIG. 8, taken along line 9-9 of FIG. 8A;
FIG. 10 depicts an exploded perspective view of the tip member of FIG. 3 and a third flexible circuit, with a distal portion of fourth flexible circuit in a pre-folded configuration;
FIG. 10A depicts a perspective view of the tip member of FIG. 3 with the third flexible circuit of FIG. 10 inserted, and with a portion of the tip member in cross-section;
FIG. 11 depicts a cross-section end view of the tip member and third flexible circuit of FIG. 10, taken along line 11-11 of FIG. 10A;
FIG. 12 depicts an exploded perspective view of the tip member of FIG. 3 and a fourth flexible circuit, with a distal portion of fourth flexible circuit in a pre-folded configuration;
FIG. 12A depicts another exploded perspective view of the tip member of FIG. 3 and the fourth flexible circuit of FIG. 12, with a portion of the tip member in cross-section;
FIG. 13 depicts a cross-section end view of the tip member and fourth flexible circuit of FIG. 12, taken along line 13-13 of FIG. 12A;
FIG. 14 depicts an exploded perspective view of the tip member of FIG. 3 and a fifth flexible circuit, with a distal portion of fourth flexible circuit in a pre-folded configuration;
FIG. 14A depicts a perspective view of the tip member of FIG. 3 with the fifth flexible circuit of FIG. 14 inserted, and with a portion of the tip member in cross-section; and
FIG. 15 depicts a cross-section end view of the tip member and fifth flexible circuit of FIG. 14, taken along line 15-15 of FIG. 14A.

### DETAILED DESCRIPTION FOR MODES OF CARRYING OUT THE INVENTION

The following description of certain examples of the invention should not be used to limit the scope of the present invention. The drawings, which are not necessarily to scale, depict selected embodiments and are not intended to limit the scope of the invention. The detailed description illustrates by way of example, not by way of limitation, the principles of the invention. Other examples, features, aspects, embodiments, and advantages of the invention will become apparent to those skilled in the art from the following description, which is by way of illustration, one of the best modes contemplated for carrying out the invention. As will be realized, the invention is capable of other different or equivalent aspects, all without departing from the invention. Accordingly, the drawings and descriptions should be regarded as illustrative in nature and not restrictive.

Any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%. In addition, as used herein, the terms "patient," "host," "user," and "subject" refer to any human or animal subject and are not intended to limit the systems or methods to human use, although use of the subject invention in a human patient represents a preferred embodiment.

### I. Overview of Example of Ablation Catheter System

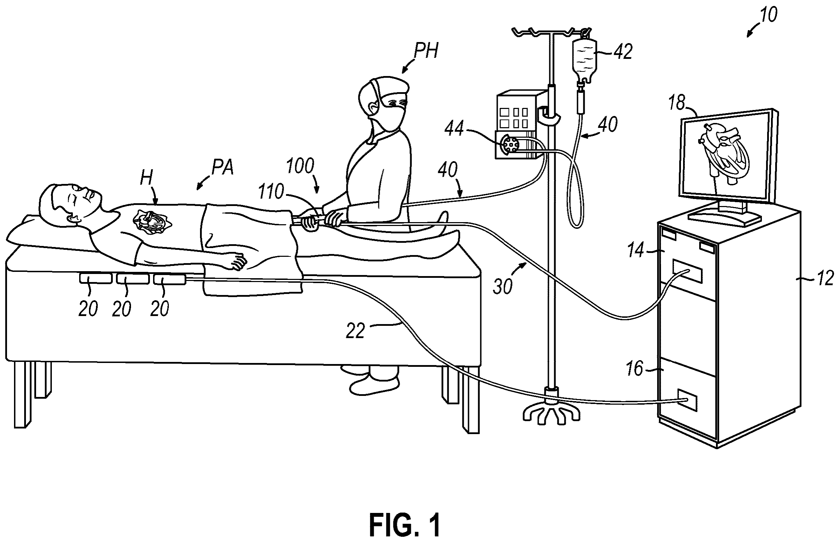

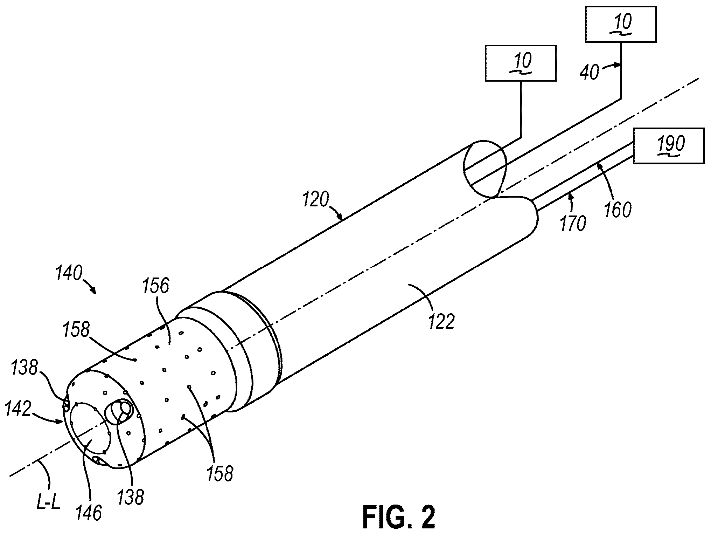

FIG. 1 shows an example of a medical procedure and associated components of a cardiac ablation catheter system that may be used to provide cardiac ablation as referred to above. In particular, FIG. 1 shows a physician (PH) grasping a handle (110) of a catheter assembly (100), with an end effector (140) of a catheter (120) (shown in FIG. 2 but not shown in FIG. 1) of catheter assembly (100) disposed in a patient (PA) to ablate tissue in or near the heart (H) of the patient (PA). As used herein, the term "ablate" is intended to include radio-frequency ablation, irreversible electroporation, or any other suitable ablation modality. Catheter assembly (100) includes handle (110), catheter (120) extending distally from handle (110), and end effector (140) located at a distal end of catheter (120).

As will be described in greater detail below, end effector (140) of the present example is operable to deliver electrical energy to targeted tissue sites, provide EP mapping functionality, track external forces imparted on end effector (140), track the location of end effector (140), and disperse irrigation fluid. User input feature (190) is configured to deflect end effector (140) and a distal portion of catheter (120) away from a central longitudinal axis (L-L).

As shown in FIG. 2, catheter (120) includes an elongate flexible sheath (122), with end effector (140) being disposed at a distal end of sheath (122). End effector (140) and various components that are contained in sheath (122) will be described in greater detail below. Catheter assembly (100) is coupled with a guidance and drive system (10) via a cable (30). Catheter assembly (100) is also coupled with a fluid source (42) via a fluid conduit (40). A set of field generators (20) are positioned underneath the patient (PA) and are coupled with guidance and drive system (10) via another cable (22). Field generators (20) are merely optional.

Guidance and drive system (10) of the present example includes a console (12) and a display (18). Console (12) includes a first driver module (14) and a second driver module (16). First driver module (14) is coupled with catheter assembly (100) via cable (30). In some variations, first driver module (14) is operable to receive EP mapping signals obtained via microelectrodes (138) of end effector (140) as described in greater detail below. Console (12) includes a processor (not shown) that processes such EP mapping signals and thereby provides EP mapping as is known in the art.

First driver module (14) of the present example is further operable to provide RF power to a distal tip member (142) of end effector (140), as will be described in greater detail below, to thereby ablate tissue. Second driver module (16) is coupled with field generators (20) via cable (22). Second driver module (16) is operable to activate field generators (20) to generate an alternating magnetic field around the heart (H) of the patient (PA). For instance, field generators (20) may include coils that generate alternating magnetic fields in a predetermined working volume that contains the heart (H).

First driver module (14) is also operable to receive position indicative signals from a navigation sensor assembly (not shown) in end effector (140). In such versions, the processor of console (12) is also operable to process the position indicative signals from navigation sensor assembly to thereby determine the position of end effector (140) within the patient (PA). By way of example only, the navigation sensor assembly may include one or more coils that are operable to generate signals that are indicative of the position and orientation of end effector (140) within the patient (PA). The coils may be configured to generate electrical signals in response to the presence of an alternating electromagnetic field generated by field generators (20). Other components and techniques that may be used to generate real-time position data associated with end effector (140) may include wireless triangulation, acoustic tracking, optical tracking, inertial tracking, and the like. Alternatively, end effector (140) may lack a navigation sensor assembly.

Display (18) is coupled with the processor of console (12) and is operable to render images of patient anatomy. Such images may be based on a set of preoperatively or intraoperatively obtained images (e.g., a CT or MRI scan, 3-D map, etc.). The views of patient anatomy provided through display (18) may also change dynamically based on signals from navigation sensor assembly of end effector (140). For instance, as end effector (140) of catheter (120) moves within the patient (PA), the corresponding position data from navigation sensor assembly may cause the processor of console (12) to update the patient anatomy views in display (18) in real time to depict the regions of patient anatomy around end effector (140) as end effector (140) moves within the patient (PA). Moreover, the processor of console (12) may drive display (18) to show locations of aberrant conductive tissue sites, as detected via electrophysiological (EP) mapping with end effector (140) or as otherwise detected (e.g., using a dedicated EP mapping catheter, etc.). The processor of console (12) may also drive display (18) to superimpose the current location of end effector (140) on the images of the patient's anatomy, such as by superimposing an illuminated dot, a crosshair, a graphical representation of end effector (140), or some other form of visual indication.

Fluid source (42) of the present example includes a bag containing saline or some other suitable irrigation fluid. Conduit (40) includes a flexible tube that is further coupled with a pump (44), which is operable to selectively drive fluid from fluid source (42) to catheter assembly (100). Such irrigation fluid may be expelled through openings (158) of distal tip member (142) of end effector (140). Such irrigation may be provided in any suitable fashion as will be apparent to those skilled in the art in view of the teachings herein.

### II. Example of End Effector of Catheter Assembly

FIGS. 2-4 show examples of components of end effector (140), and other components of the distal portion of catheter (120), in greater detail. End effector (140) includes a distal tip member (142) and a distal portion of a flexible circuit (200) having a thermocouple assembly (210). A pair of push-pull cables (160, 170) extend along the length of catheter (120) to reach end effector (140). Push-pull cables (160, 170) enable the physician (PH) to selectively deflect end effector (140) laterally away from the central longitudinal axis (L-L), thereby enabling the physician (PH) to actively steer end effector (140) within the patient (PA). Various mechanisms that may be used to drive push-pull cables (160, 170) in a simultaneous, longitudinally-opposing fashion will be apparent to those skilled in the art in view of the teachings herein. Flexible sheath (122) surrounds a portion of push-pull cables (160, 170) and a portion of flexible circuit (200).

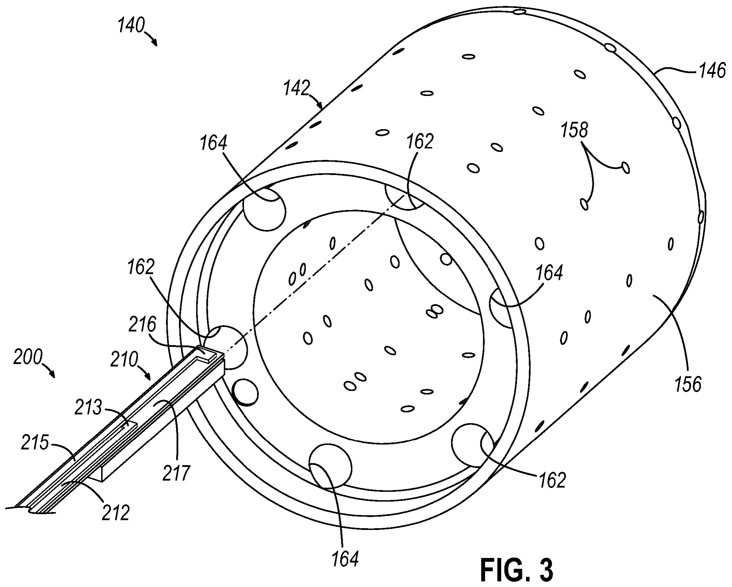

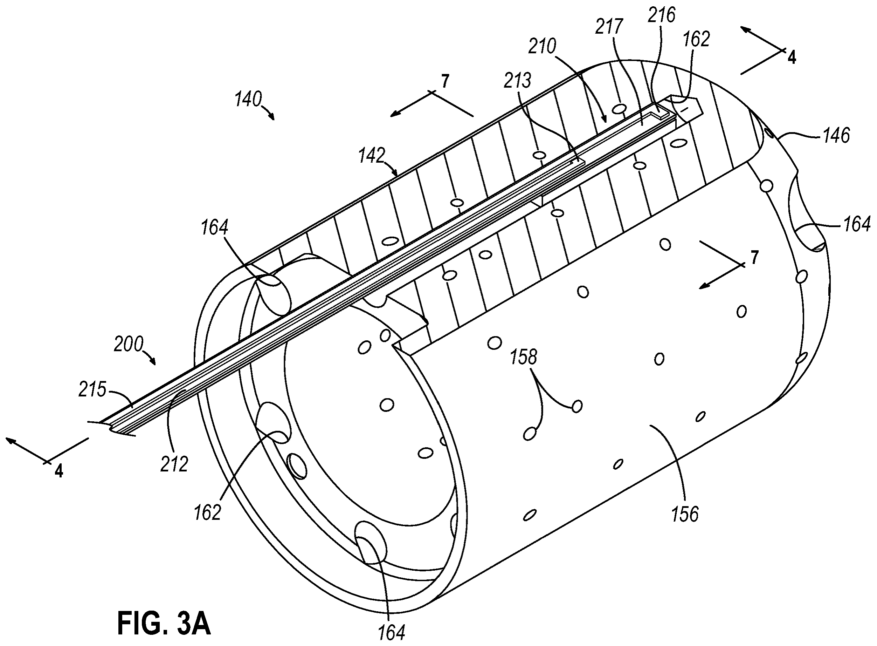

As shown in FIGS. 3-4, distal tip member (142) of the present example is electrically conductive and includes a cylindraceous body (156) with a dome tip (146). A plurality of openings (158) are formed through cylindraceous body (156) and are in communication with the hollow interior of distal tip member (142). Openings (158) thus allow irrigation fluid to be communicated from the interior of distal tip member (142) out through cylindraceous body (156). Cylindraceous body (156) and dome tip (146) are also operable to apply RF electrical energy to tissue to thereby ablate the tissue. Such RF electrical energy may be communicated from first driver module (14) to cylindraceous body (156) via cable (30) and any number of electrically conductive components (not shown) that are interposed between cylindraceous body (156) and cable (30) (e.g., wires, traces in flex circuits, etc.).

As best seen in FIGS. 3-4, distal tip member (142) of the present example also includes a first set of bores (162) and a second set of bores (164). Bores (162, 164) are formed in cylindraceous body (156) and are angularly spaced apart from each other about the central longitudinal axis (L-L) in an alternating arrangement. Bores (162) distally terminate within cylindraceous body (156), such that bores (162) constitute blind bores that do not extend through dome tip (146). Bores (164) extend fully through cylindraceous body (156) and dome tip (146). Bores (162) receive thermocouple assemblies (210), which are configured to provide temperature sensing capabilities, as will be described in greater detail below. Bores (164) receive EP mapping electrodes (138). EP mapping microelectrodes (138) are configured to pick up electrical potentials from tissue that contacts EP mapping microelectrodes (138). First driver module (14) may process the EP mapping signals and provide the physician (PH) with corresponding feedback indicating the locations of aberrant electrical activity in accordance with the teachings of various references cited herein.

As also noted above, catheter assembly (100) is configured to enable irrigation fluid to be communicated from fluid source (42) to catheter (120) via fluid conduit (40), thereby providing expulsion of the irrigation fluid via openings (158) of distal tip member (142). In some versions, the fluid path for the irrigation fluid includes an irrigation tube (not shown) that is positioned within sheath (122) and is coupled with fluid conduit (40) (e.g., at handle (110) of catheter assembly (100)). Such an irrigation tube may extend along the length of catheter (120) to reach end effector (140). In some versions, irrigation fluid may be communicated from the distal end of the irrigation tube through a central passageway formed by aligned central apertures of the features described above, ultimately reaching the hollow interior of distal tip member (142) prior to flowing out from openings (158).

### III. First Example of a Flexible Circuit

As noted above, some versions of thermocouples may be difficult to manufacture, may be difficult to position for accurate temperature readings because of their small size, and/or may tend to provide undesirably high scrap rates during manufacturing. However, thermocouple assemblies (210) of the present example, and their arrangement within end effector (140), may tend to alleviate such issues. In other words, the configuration and arrangement of thermocouple assemblies (210) within end effector (140) may tend to provide faster and/or more cost-effective manufacturing as compared to conventional thermocouples, may tend to provide easier positioning for accurate temperature readings as compared to conventional thermocouples, and/or may tend to provide lower scrap rates during manufacture as compared to conventional thermocouples.

FIGS. 3-4 show a distal end of a flexible circuit (200), which includes thermocouple assembly (210), positioned inside of bore (162) of distal tip member (142). While only one flexible circuit (200) is shown, three flexible circuits (200) (or any other suitable number of flexible circuits (200)) may be provided, with the thermocouple assembly (210) of each flexible circuit (200) being positioned within a respective bore (162) of distal tip member (142). In some versions, three discrete flexible circuits (200) may extend separately from each other along the length of sheath (122). In some other versions, three flexible circuits (200) may join together within sheath (122) (e.g., near end effector (140)), or within end effector (140), to form a unitary flex circuit, which may then extend along the remaining proximal length of sheath (122).

Thermocouple assembly (210) of the present example includes an electrically conductive first trace (212), an electrically conductive second trace (215), and an electrically conductive third trace (219). First and second traces (212, 215) extend along an upper surface of an electrically insulating substrate layer (217). By way of example only, substrate layer (217) may comprise polyimide and/or any other suitable material(s). Third trace (219) extends along a lower surface of substrate layer (217). Traces (212, 215, 219) and substrate layer (217) extend along the length of sheath (122). These features of flexible circuit (200) are flexible to allow catheter (120) to readily flex during operation by the physician (PH), such as when catheter (120) traverses a tortuous path within the patient (PA) and when the physician (PH) drives deflection of the distal region of catheter (120), etc.

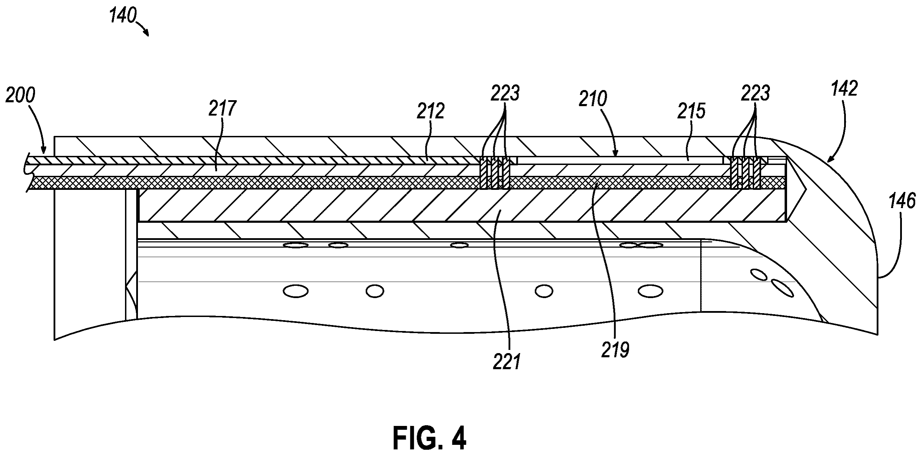

The distal end of first trace (212) includes a first thermal contact (213), while the distal end of second trace (215) includes a second thermal contact (216). Thermocouple assembly (210) is sized to fit inside bore (162), with first and second thermal contacts (213, 216) facing radially outwardly. As shown in FIG. 4, proper positioning may be achieved once thermocouple assembly (210) is fully inserted into bore (162), with the distal end of thermocouple assembly (210) being just proximal to dome tip (146).

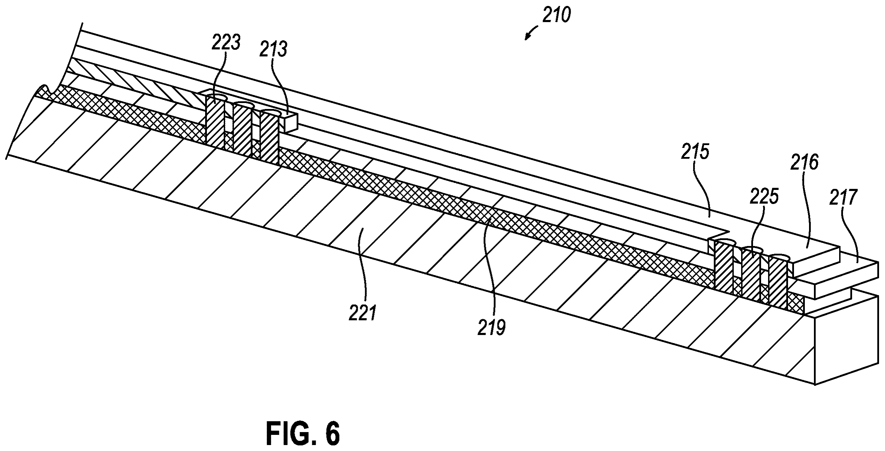

As shown in FIGS. 4 and 6-7, first thermal contact (213) is electrically coupled with third trace (219) via junctions (223); while second thermal contact (216) is electrically coupled with third trace (219) via junctions (225). Junctions (223, 225) pass through the full thickness of insulating layer (217). In the present example, each of first trace (212) (including first thermal contact (213)) and second trace (215) (including second thermal contact (216)) comprises a first conductive material (e.g., copper, etc.) while third trace (219) comprises a second conductive material (e.g., constantan or copper-nickel alloy, etc.). Due to the dissimilarity of conductive materials, changes in the temperatures of junctions (223, 225) will provide a temperature gradient between first trace (212) and third trace (219), and between second trace (215) and third trace (219), respectively. These temperature gradients may produce a thermoelectric voltage, which is proportional to the temperature difference between the distal end of first and second traces (212, 215) and the corresponding regions of third trace (219). Such thermoelectric voltages may be read by a processor of console (12) as will be described in greater detail below. It should also be understood that these changes in the temperatures of junctions (223, 225) may be caused by changes in the temperatures of thermal contacts (213, 216), which may be induced by heating or cooling of distal tip member (142).

While both thermal contacts (213, 216) are electrically coupled with third trace (219) via respective junctions (223, 225) in this example, some other versions may provide a fourth trace that is positioned on the underside of insulating layer (217) and discrete from third trace (219). In some such variations first thermal contact (213) is electrically coupled with third trace (219) via junction (223); while second thermal contact (216) is electrically coupled with the fourth trace via junction (225). In such variations, the fourth trace may comprise an electrically conductive material that differs from the electrically conductive material of second trace (215).

In some versions, at least a portion of each trace (212, 215) and thermal contact (213, 216) is coated with an electrically insulating coating (not shown) to thereby electrically insulate traces (212, 215) and thermal contacts (213, 216) relative to distal tip member (142). Such an electrically insulating coating may nevertheless be thermally conductive, such that thermal contacts (213, 216) are in thermal communication with distal tip member (142). Specifically, thermal contacts (213, 216) may thermally contact the inner sidewall of bore (162) without being in electrical communication with the inner sidewall of bore (162), while maintaining thermal communication with the inner sidewall of bore (162). Additional features of thermocouple assembly (210) that may further promote thermal coupling of thermal contacts (213, 216) with distal tip member (142) will be described in greater detail below.

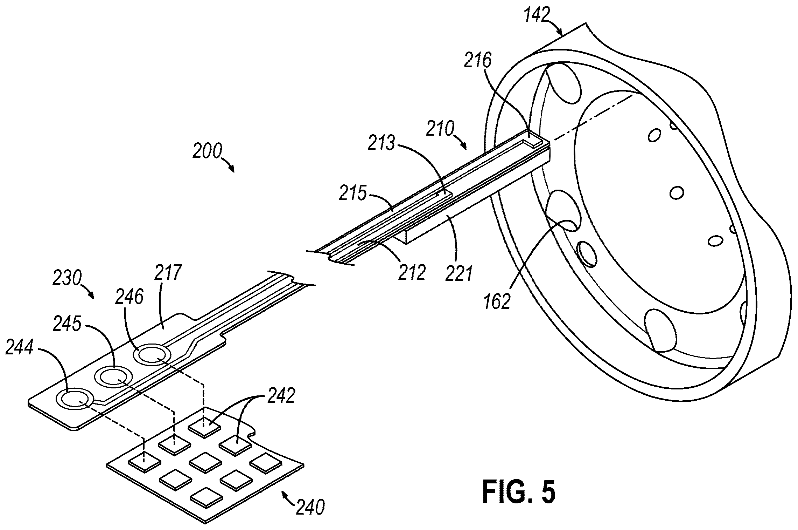

As shown in FIG. 5, a proximal end (230) of flexible circuit (200) includes solder pads (244, 245, 246) arranged in a straight row. First trace (212) is in electrical communication with solder pad (244), second trace (215) is in electrical communication with solder pad (246), and third trace (216) is in electrical communication with solder pad (245). A printed circuit board (PCB) (240) is coupled with proximal end (230) of flexible circuit (200). Specifically, PCB (240) includes a set of solder pads (242) that are electrically coupled with respective solder pads (244, 245, 246) of flexible circuit (200). PCB (240) may be positioned in end effector (140), elongate flexible sheath (122), or handle (110). PCB (240) is further electrically coupled with a processor of console (12) via cable (30) and any number of electrically conductive components (not shown) that are interposed between PCB (240) and cable (30) (e.g., wires, traces in flex circuits, etc.). PCB (240), cable (30), and intervening electrically conductive components thus provide a pathway for communication of voltages from thermocouple assembly (210) to the processor of console (12). This allows the processor of console (12) to process temperature-indicative data picked up via thermocouple assembly (210) to thereby sense the temperature of end effector (140), which may further indicate the temperature of tissue contacting end effector (140).

End effector (140) may include several separate thermocouple assemblies (210) angularly spaced apart from each other in distal tip member (142), with each thermocouple assembly (210) being positioned in a respective bore (162). Each thermocouple assembly (210) may be incorporated into respective discrete flexible circuits (200). Each flexible circuit (200) may also be coupled with a respective PCB (240). Alternatively, each flexible circuit (200) may be attached to a common PCB (240), where the PCB (240) has a number of rows of PCB pads (242) that complements the number of thermocouple assemblies (210). By way of example only, should PCB (240) be positioned in either handle (110) or a proximal portion of elongate flexible sheath (122), flexible circuit (200) may extend to 2 meters or more. Each flexible circuit (200) may be the same length as any other flexible circuit (200). By PCB (240) being in separate electrical communication with each thermocouple assembly (210) of each flexible circuit (200), PCB (240) may be capable of differentiating temperature readings across distal tip member (142) and communicating these readings to the processor of the console (14). Flexible circuit (200) may also include a reference lead (not shown) extending from solder pads (245) to thermocouple assembly (210), which may act to detect signal noise that can subsequently be removed or considered when evaluating thermocouple assembly (210) voltage readings.

PCB (240) may comprise a flat single or multilayer circuit board such that flexible circuits (200) twist relative to each other to thereby attach to distal tip member (142) while also angularly positioning each thermocouple assembly (210) radially outwards as described above. Given the extended length of flexible circuits (200) relative to the diameter of elongate flexible sheath (122) in the present example, bending of elongate flexible sheath (122) may tend to result in some degree of pulling of each flexible circuit (200) relative to distal tip member (142). Adhesive holding thermocouple assembly (210) inside of bore (162) may be sufficient to resist pulling of thermocouple assembly (210) from bore when flexible circuit (200) is pulled due to bending of sheath (122). To further ensure little to no pulling at thermocouple assembly (210), flexible circuits (200) may also be longer than the length between PCB (240) and bore (162) to provide slack that accommodates flexing-induced changes in the effective length of each flexible circuit (200). In other words, flexible circuit (200) may be bunched along the length from PCB (240) to bore (162).

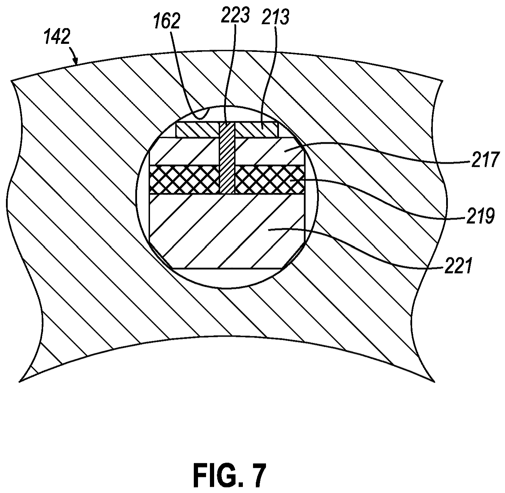

As shown in FIGS. 4-7, the region of flexible circuit (200) forming thermocouple assembly (210) further includes an additional electrically insulating spacer layer (221), which is positioned under third trace (219). Third trace (219) is thus interposed between layers (217, 221). By way of example only, spacer layer (221) may comprise polyimide and/or any other suitable material(s). Spacer layer (221) is substantially thicker than substrate layer (217) in this example. With the presence of spacer layer (221), thermocouple assembly (210) is oversized relative to bore (162) such that at least a portion of thermocouple assembly (210) must compress or otherwise deform for thermocouple assembly (210) to fit within bore (162). In the present example, spacer layer (221) is compressible, such that outer corners of spacer layer (221) deform against the inner sidewall of bore (162) as shown in FIG. 7. Spacer layer (221) may be resiliently biased toward a non-deformed state, such that the compression of spacer layer (221) within bore (162) may tend to provide firm securement of thermocouple assembly (210) within bore (162). Moreover, such compression of spacer layer (221) within bore (162) may tend to urge the upper portion of thermocouple assembly (210) radially outwardly relative to the central longitudinal axis (L-L). This effect may in turn minimize the distance between thermal contacts (213, 216) and the outer surface of cylindraceous body (156), which may maximize the sensitivity of thermal contacts (213, 216) to the temperature of cylindraceous body (156) and tissue in contact with cylindraceous body (156). This may in turn maximize the sensitivity of junctions (223, 225) to the temperature of cylindraceous body (156) and tissue in contact with cylindraceous body (156). Spacer layer (221) may thus promote thermal conductivity between thermocouple assembly (210) and cylindraceous body (156).

While thermocouple assembly (210) may be suitably secured within bore (162) through friction in some cases as described above, the securement of thermocouple assembly (210) within bore (162) may be further supplemented with adhesive. By way of example only, such adhesive may be provided via thermoplastic polyurethane elastomer that is introduced into bore (162) after thermocouple assembly (210) has been fully seated in bore (162). By way of further example only, such thermoplastic polyurethane elastomer may be doped with diamond powder and may be cured with ultraviolet light. Alternatively, any other suitable kind of adhesive may be used, and any other suitable process may be used to bond thermocouple assembly (210) relative to distal tip member (142).

### IV. Second Example of a Flexible Circuit

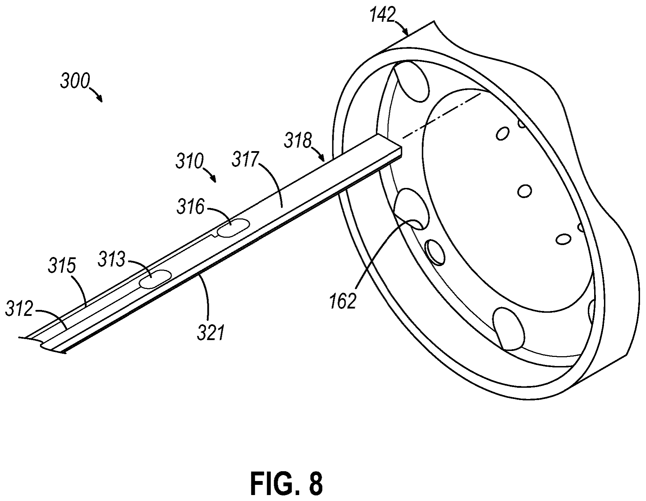

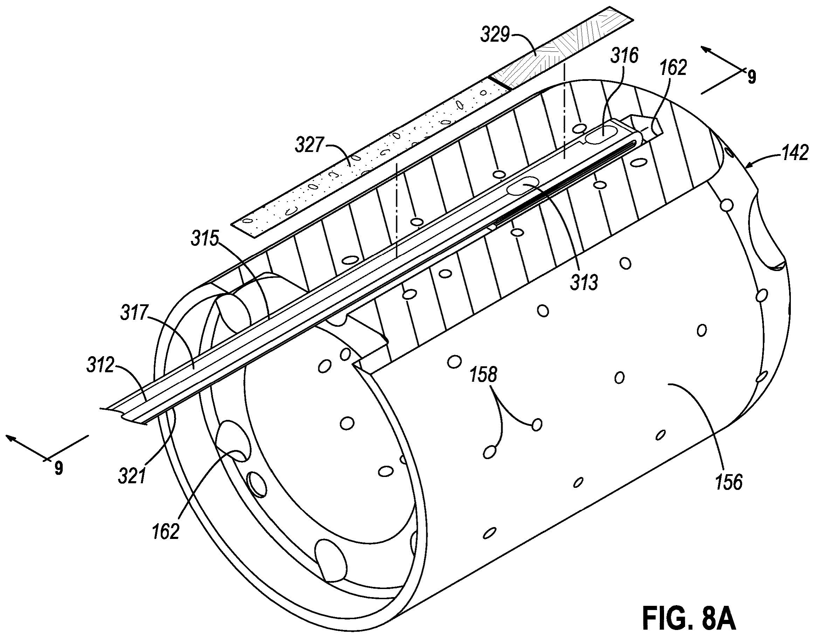

FIGS. 8-9 show a second example of a flexible circuit (300) that may be substantially identical in form and function to flexible circuit (200) except for any differences that are described below. Flexible circuit (200) may thus be substituted with flexible circuit (300). Like flexible circuit (200), flexible circuit (300) of this example includes a thermocouple assembly (310) that includes an electrically conductive first trace (312), an electrically conductive second trace (315), and an electrically conductive third trace (321). First and second traces (312, 315) extend along an upper surface of an electrically insulating substrate layer (317). By way of example only, substrate layer (317) may comprise polyimide and/or any other suitable material(s). Third trace (321) extends along a lower surface of substrate layer (317). Traces (312, 315, 321) and substrate layer (317) extend along the length of sheath (122).

The distal end of first trace (312) includes a first thermal contact (313), while the distal end of second trace (315) includes a second thermal contact (316). First thermal contact (313) is electrically coupled with third trace (321) via junctions (323); while second thermal contact (316) is electrically coupled with third trace (321) via junctions (325). Junctions (323, 325) pass through the full thickness of substrate layer (317). In the present example, each of first trace (312) (including first thermal contact (313)) and second trace (315) (including second thermal contact (316)) comprises a first conductive material (e.g., copper, etc.) while third trace (321) comprises a second conductive material (e.g., constantan or copper-nickel alloy, etc.). Due to the dissimilarity of conductive materials, changes in the temperatures of junctions (323, 325) will provide a temperature gradient between first trace (312) and third trace (321), and between second trace (315) and third trace (321), respectively. These temperature gradients may produce thermoelectric voltages. Thermocouple assembly (310) may thus provide temperature sensing in a way similar to that described above with respect to thermocouple assembly (210).

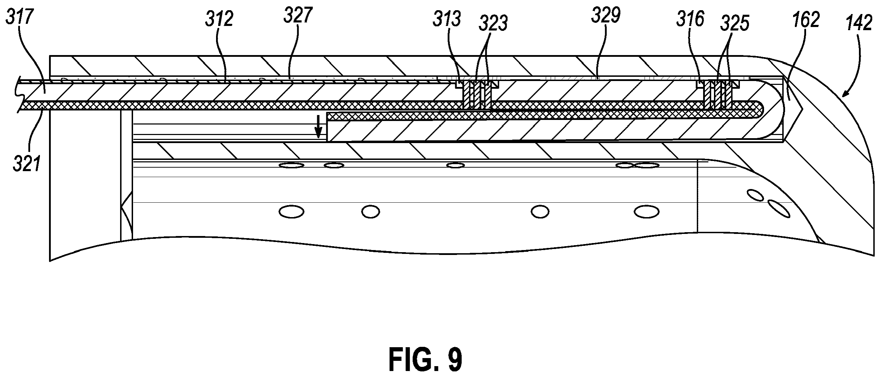

As shown in FIGS. 8A and 9, an electrically insulating coating (329) may be positioned over thermal contacts (313, 316) and at least a portion of traces (312, 315). In some cases, coating (329) extends further along the length of traces (312, 315). Coating (329) electrically insulates traces (312, 315) and thermal contacts (313, 316) relative to distal tip member (142). Coating (329) is nevertheless thermally conductive, such that thermal contacts (313, 316) are in thermal communication with distal tip member (142). Specifically, thermal contacts (313, 316) may thermally contact the inner sidewall of bore (162) without being in electrical communication with the inner sidewall of bore (162), while maintaining thermal communication with the inner sidewall of bore (162).

Flexible circuit (300) of this example further includes a distal portion (318) of substrate layer (317) that extends distally beyond thermal contacts (313, 316). As shown in FIG. 8A, distal portion (318) may be folded in a proximal direction before thermocouple assembly (310) is inserted into bore (162). The folded-over distal portion (318) may act as a spring, similar to the partially compressed spacer layer (221) of flexible circuit (200), to resiliently bias thermocouple assembly (310) radially outwardly relative to the central longitudinal axis (L-L). This effect may in turn minimize the distance between thermal contacts (313, 316) and the outer surface of cylindraceous body (156), which may maximize the sensitivity of thermal contacts (313, 316) to the temperature of cylindraceous body (156) and tissue in contact with cylindraceous body (156). This may in turn maximize the sensitivity of junctions (323, 325) to the temperature of cylindraceous body (156) and tissue in contact with cylindraceous body (156). Folded-over distal portion (318)) may thus promote thermal conductivity between thermocouple assembly (310) and cylindraceous body (156).

Further, folded-over distal portion (318) may tend to provide securement of thermocouple assembly (310) within bore (162) by expanding the effective cross-sectional profile of thermocouple assembly (310). In some cases, the resilient bias of folded-over distal portion (318) urges distal portion (318) toward a straight/unfolded configuration, such that distal portion (318) bears against the inner sidewall of bore (162), thereby enhancing friction to secure thermocouple assembly (310) within bore (162).

Positioning and securement of thermocouple assembly (310) within bore (162) may be further supplemented with an adhesive (327) to thus adhere flexible circuit (300) relative to distal tip member (142). In some cases where an adhesive is used, and while not shown in the present drawings, at least some of the adhesive may occupy a space formed between folded-over distal portion (318) and the underside of third trace (321) and substrate layer (317) (or the underside of an additional electrically insulating layer (not shown) that is positioned under third trace (321)). By way of example only, such adhesive may be provided via thermoplastic polyurethane elastomer that is introduced into bore (162) after thermocouple assembly (310) has been fully seated in bore (162). By way of further example only, such thermoplastic polyurethane elastomer may be doped with diamond powder and may be cured with ultraviolet light. Alternatively, any other suitable kind of adhesive may be used, and any other suitable process may be used to bond thermocouple assembly (310) relative to distal tip member (142).

### V. Third Example of a Flexible Circuit

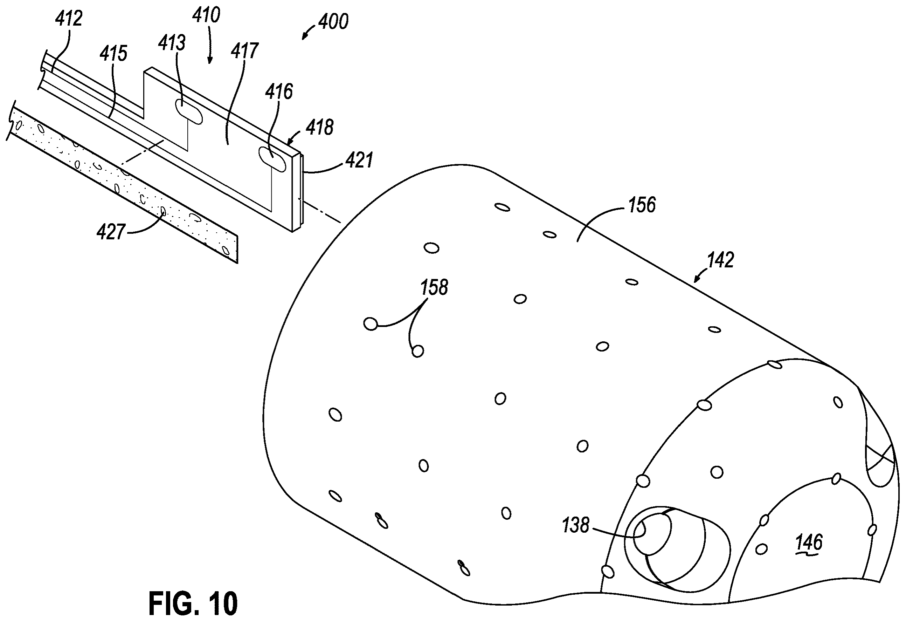

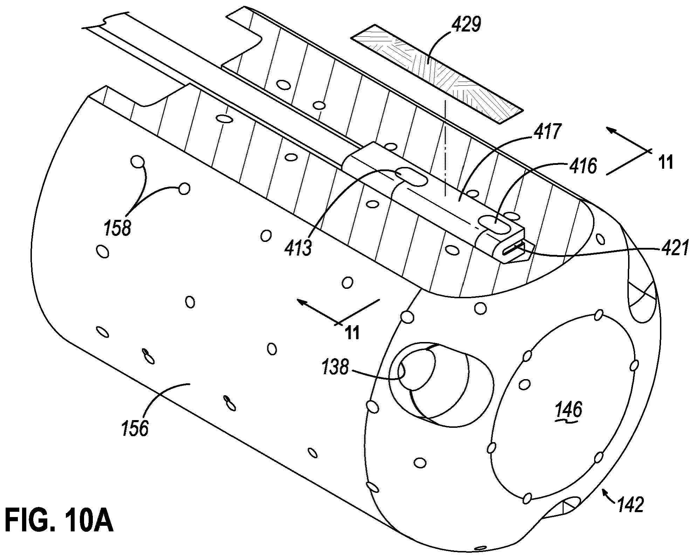

FIGS. 10-11 show a third example of a flexible circuit (400) that may be substantially identical in form and function to flexible circuits (200, 300) except for any differences that are described below. Flexible circuit (200) may thus be substituted with flexible circuit (400). Like flexible circuit (200), flexible circuit (400) of this example includes a thermocouple assembly (410) that includes an electrically conductive first trace (412), an electrically conductive second trace (415), and an electrically conductive third trace (421). First and second traces (412, 415) extend along an upper surface of an electrically insulating substrate layer (417). By way of example only, substrate layer (417) may comprise polyimide and/or any other suitable material(s). Third trace (421) extends along a lower surface of substrate layer (417). Traces (412, 415, 421) and substrate layer (417) extend along the length of sheath (122).

The distal end of first trace (412) includes a first thermal contact (413), while the distal end of second trace (415) includes a second thermal contact (416). First thermal contact (413) is electrically coupled with third trace (421) via junctions (not shown); while second thermal contact (416) is electrically coupled with third trace (421) via junctions (425). Junctions (425) pass through the full thickness of substrate layer (417). In the present example, each of first trace (412) (including first thermal contact (413)) and second trace (415) (including second thermal contact (416)) comprises a first conductive material (e.g., copper, etc.) while third trace (421) comprises a second conductive material (e.g., constantan or copper-nickel alloy, etc.). Due to the dissimilarity of conductive materials, changes in the temperatures of junctions (425) will provide a temperature gradient between first trace (412) and third trace (421), and between second trace (415) and third trace (421), respectively. These temperature gradients may produce thermoelectric voltages. Thermocouple assembly (410) may thus provide temperature sensing in a way similar to that described above with respect to thermocouple assembly (210).

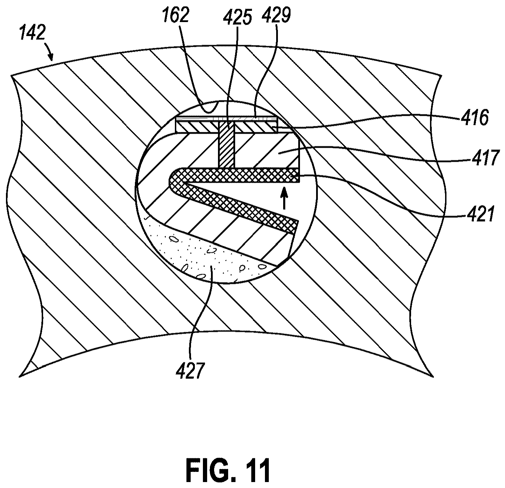

As shown in FIGS. 10A and 11, an electrically insulating coating (429) may be positioned over thermal contacts (413, 416) and at least a portion of traces (412, 415). In some cases, coating (429) extends further along the length of traces (412, 415). Coating (429) electrically insulates traces (412, 415) and thermal contacts (413, 416) relative to distal tip member (142). Coating (429) is nevertheless thermally conductive, such that thermal contacts (413, 416) are in thermal communication with distal tip member (142). Specifically, thermal contacts (413, 416) may thermally contact the inner sidewall of bore (162) without being in electrical communication with the inner sidewall of bore (162), while maintaining thermal communication with the inner sidewall of bore (162).

Flexible circuit (400) of this example further includes a lateral portion (418) of substrate layer (417) that projects laterally relative to the adjacent portion of substate layer (417). Thermal contacts (413, 416) are positioned on lateral portion (418), with distal portions of traces (412, 415) extending along lateral portion (418) to reach thermal contacts (413, 416). Third trace (421) also extends along lateral portion (418). As shown in FIG. 10A-11, lateral portion (418) is folded over to position lateral portion (418) under the adjacent region of substrate layer (417). The folded-over lateral portion (418) may act as a spring, similar to the partially compressed spacer layer (221) of flexible circuit (200) and folded-over distal portion (318) of flexible circuit (300), to resiliently bias thermocouple assembly (410) radially outwardly relative to the central longitudinal axis (L-L). This effect may in turn minimize the distance between thermal contacts (413, 416) and the outer surface of cylindraceous body (156), which may maximize the sensitivity of thermal contacts (413, 416) to the temperature of cylindraceous body (156) and tissue in contact with cylindraceous body (156). This may in turn maximize the sensitivity of junctions (425) to the temperature of cylindraceous body (156) and tissue in contact with cylindraceous body (156). Folded-over lateral portion (418)) may thus promote thermal conductivity between thermocouple assembly (410) and cylindraceous body (156).

Further, folded-over lateral portion (418) may tend to provide securement of thermocouple assembly (410) within bore (162) by expanding the effective cross-sectional profile of thermocouple assembly (410). In some cases, the resilient bias of folded-over lateral portion (418) urges lateral portion (418) toward a flat/unfolded configuration, such that lateral portion (418) bears against the inner sidewall of bore (162), thereby enhancing friction to secure thermocouple assembly (410) within bore (162).

Positioning and securement of thermocouple assembly (410) within bore (162) may be further supplemented with an adhesive (427) to thus adhere thermocouple assembly (410) relative to distal tip member (142). In some cases where an adhesive is used, and while not shown in the present drawings, at least some of the adhesive may occupy a space formed between the folded-over lateral portion (418) and the underside of third trace (421) and substrate layer (417) (or the underside of an additional electrically insulating layer (not shown) that is positioned under third trace (421)). By way of example only, such adhesive may be provided via thermoplastic polyurethane elastomer that is introduced into bore (162) after thermocouple assembly (410) has been fully seated in bore (162). By way of further example only, such thermoplastic polyurethane elastomer may be doped with diamond powder and may be cured with ultraviolet light. Alternatively, any other suitable kind of adhesive may be used, and any other suitable process may be used to bond thermocouple assembly (410) relative to distal tip member (142).

### VI. Fourth Example of a Flexible Circuit

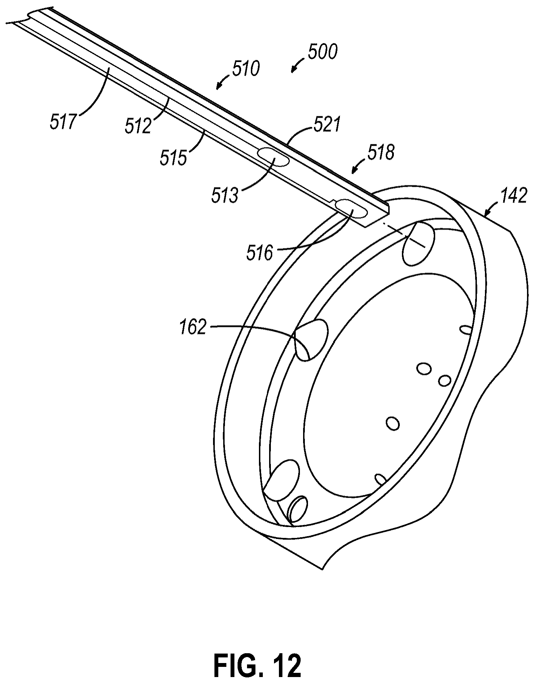

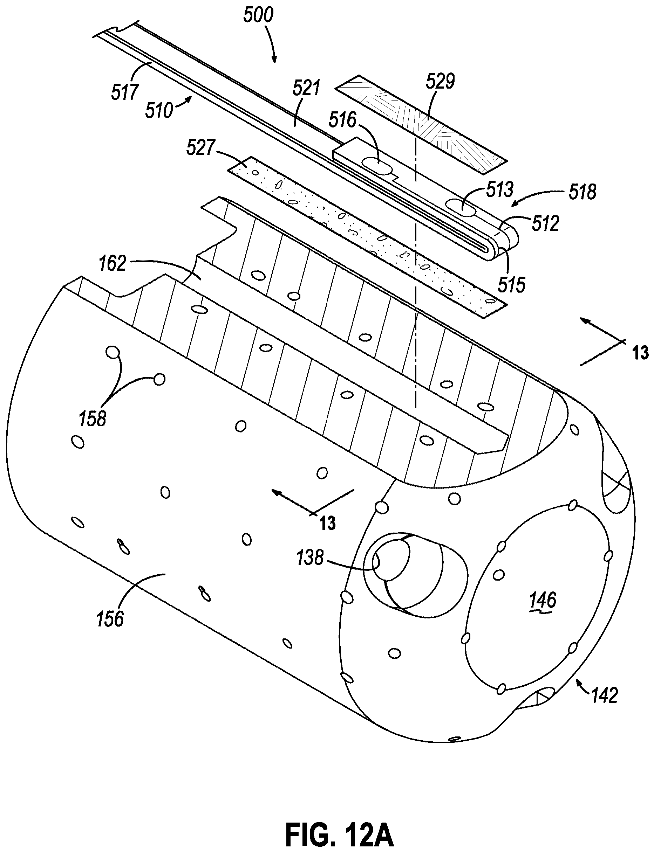

FIGS. 12-13 show a fourth example of a flexible circuit (500) that may be substantially identical in form and function to flexible circuits (200, 300, 400) except for any differences that are described below. Flexible circuit (200) may thus be substituted with flexible circuit (500). Like flexible circuits (200, 300, 400), flexible circuit (500) of this example includes a thermocouple assembly (510) that includes an electrically conductive first trace (512), an electrically conductive second trace (515), and an electrically conductive third trace (521). First and second traces (512, 515) extend along an upper surface of an electrically insulating substrate layer (517). By way of example only, substrate layer (517) may comprise polyimide and/or any other suitable material(s). Third trace (521) extends along a lower surface of substrate layer (517). Traces (512, 515, 521) and substrate layer (517) extend along the length of sheath (122).

The distal end of first trace (512) includes a first thermal contact (513), while the distal end of second trace (515) includes a second thermal contact (516). First thermal contact (513) is electrically coupled with third trace (521) via junctions (523); while second thermal contact (516) is electrically coupled with third trace (521) via similar junctions (not shown). Junctions (523) pass through the full thickness of substrate layer (517). In the present example, each of first trace (512) (including first thermal contact (513)) and second trace (515) (including second thermal contact (516)) comprises a first conductive material (e.g., copper, etc.) while third trace (521) comprises a second conductive material (e.g., constantan or copper-nickel alloy, etc.). Due to the dissimilarity of conductive materials, changes in the temperatures of junctions (523) will provide a temperature gradient between first trace (512) and third trace (521), and between second trace (515) and third trace (521), respectively. These temperature gradients may produce thermoelectric voltages. Thermocouple assembly (510) may thus provide temperature sensing in a way similar to that described above with respect to thermocouple assembly (210).

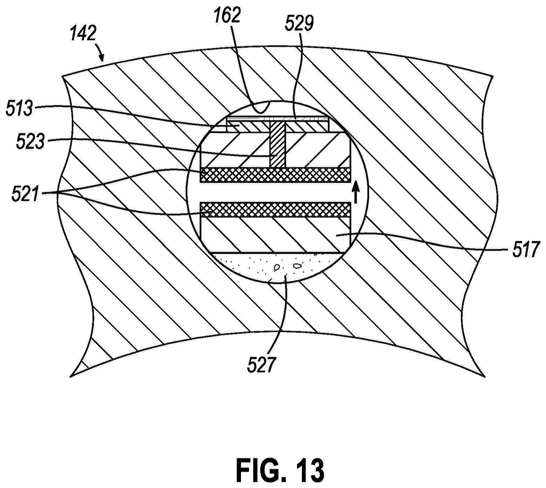

As shown in FIGS. 12A and 13, an electrically insulating coating (529) may be positioned over thermal contacts (513, 516) and at least a portion of traces (512, 515). In some cases, coating (529) extends further along the length of traces (512, 515). Coating (529) electrically insulates traces (512, 515) and thermal contacts (513, 516) relative to distal tip member (142). Coating (529) is nevertheless thermally conductive, such that thermal contacts (513, 516) are in thermal communication with distal tip member (142). Specifically, thermal contacts (513, 516) may thermally contact the inner sidewall of bore (162) without being in electrical communication with the inner sidewall of bore (162), while maintaining thermal communication with the inner sidewall of bore (162).

Thermal contacts (513, 516) are positioned on a distal portion (518) of substrate layer (517) in flexible circuit (500) of this example. As shown in FIG. 12A, distal portion (518) may be folded in a proximal direction before thermocouple assembly (510) is inserted into bore (162). The folded-over distal portion (518) may act as a spring, similar to the partially compressed spacer layer (221) of flexible circuit (200), to resiliently bias thermocouple assembly (510) radially outwardly relative to the central longitudinal axis (L-L). This effect may in turn minimize the distance between thermal contacts (513, 516) and the outer surface of cylindraceous body (156), which may maximize the sensitivity of thermal contacts (513, 516) to the temperature of cylindraceous body (156) and tissue in contact with cylindraceous body (156). This may in turn maximize the sensitivity of junctions (323, 325) to the temperature of cylindraceous body (156) and tissue in contact with cylindraceous body (156). Folded-over distal portion (518)) may thus promote thermal conductivity between thermocouple assembly (510) and cylindraceous body (156).

Further, folded-over distal portion (518) may tend to provide securement of thermocouple assembly (510) within bore (162) by expanding the effective cross-sectional profile of thermocouple assembly (510). In some cases, the resilient bias of folded-over distal portion (518) urges distal portion (518) toward a straight/unfolded configuration, such that distal portion (518) bears against the inner sidewall of bore (162), thereby enhancing friction to secure thermocouple assembly (510) within bore (162).

Positioning and securement of thermocouple assembly (510) within bore (162) may be further supplemented with an adhesive (527) to thus adhere flexible circuit (500) relative to distal tip member (142). In some cases where an adhesive is used, and while not shown in the present drawings, at least some of the adhesive may occupy a space formed between folded-over distal portion (518) and the underside of third trace (521) and substrate layer (517) (or the underside of an additional electrically insulating layer (not shown) that is positioned under third trace (521)). By way of example only, such adhesive may be provided via thermoplastic polyurethane elastomer that is introduced into bore (162) after thermocouple assembly (510) has been fully seated in bore (162). By way of further example only, such thermoplastic polyurethane elastomer may be doped with diamond powder and may be cured with ultraviolet light. Alternatively, any other suitable kind of adhesive may be used, and any other suitable process may be used to bond thermocouple assembly (510) relative to distal tip member (142).

### VII. Fifth Example of a Flexible Circuit

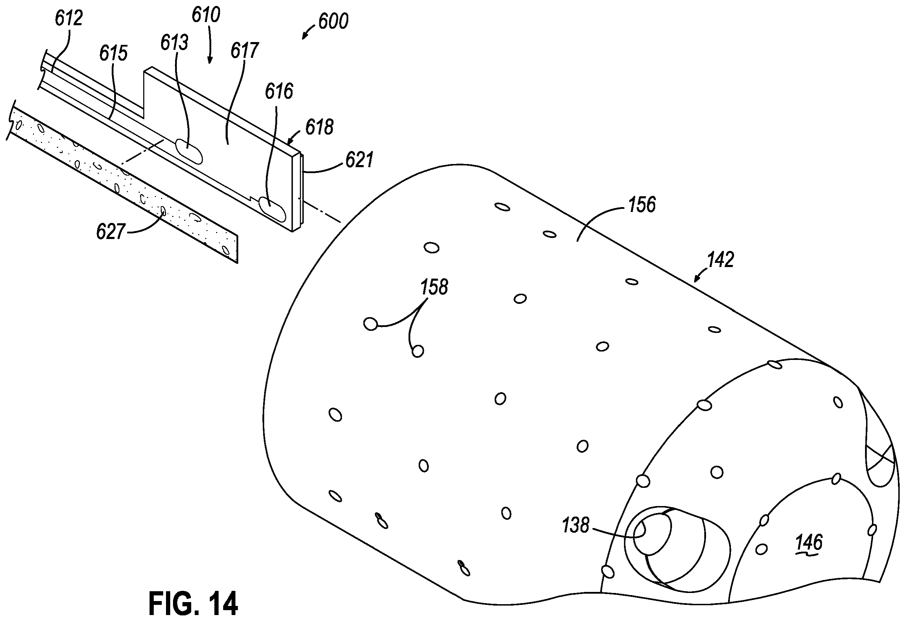

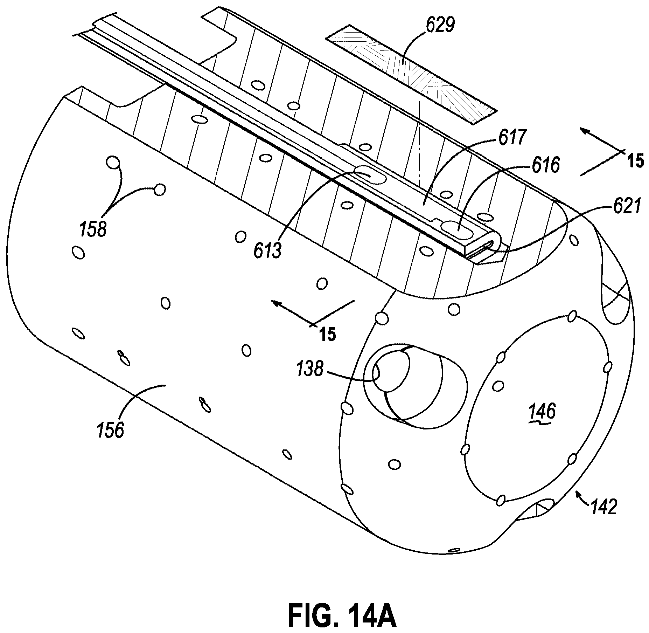

FIGS. 14-15 show a fifth example of a flexible circuit (600) that may be substantially identical in form and function to flexible circuits (200, 300, 400, 500) except for any differences that are described below. Flexible circuit (200) may thus be substituted with flexible circuit (600). Like flexible circuit (200), flexible circuit (600) of this example includes a thermocouple assembly (610) that includes an electrically conductive first trace (612), an electrically conductive second trace (615), and an electrically conductive third trace (621). First and second traces (612, 615) extend along an upper surface of an electrically insulating substrate layer (617). By way of example only, substrate layer (617) may comprise polyimide and/or any other suitable material(s). Third trace (621) extends along a lower surface of substrate layer (617). Traces (612, 615, 621) and substrate layer (617) extend along the length of sheath (122).

The distal end of first trace (612) includes a first thermal contact (613), while the distal end of second trace (615) includes a second thermal contact (616). First thermal contact (613) is electrically coupled with third trace (621) via junctions (not shown); while second thermal contact (616) is electrically coupled with third trace (621) via junctions (625). Junctions (625) pass through the full thickness of substrate layer (617). In the present example, each of first trace (612) (including first thermal contact (613)) and second trace (615) (including second thermal contact (616)) comprises a first conductive material (e.g., copper, etc.) while third trace (621) comprises a second conductive material (e.g., constantan or copper-nickel alloy, etc.). Due to the dissimilarity of conductive materials, changes in the temperatures of junctions (625) will provide a temperature gradient between first trace (612) and third trace (621), and between second trace (615) and third trace (621), respectively. These temperature gradients may produce thermoelectric voltages. Thermocouple assembly (610) may thus provide temperature sensing in a way similar to that described above with respect to thermocouple assembly (210).

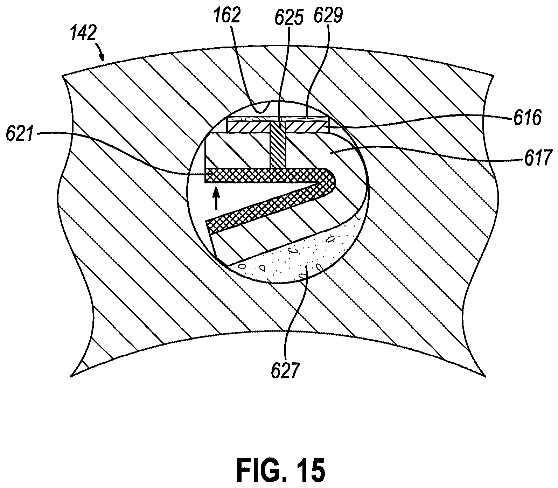

As shown in FIGS. 14A and 15, an electrically insulating coating (629) may be positioned over thermal contacts (613, 616) and at least a portion of traces (612, 615). In some cases, coating (629) extends further along the length of traces (612, 615). Coating (629) electrically insulates traces (612, 615) and thermal contacts (613, 616) relative to distal tip member (142). Coating (629) is nevertheless thermally conductive, such that thermal contacts (613, 616) are in thermal communication with distal tip member (142). Specifically, thermal contacts (613, 616) may thermally contact the inner sidewall of bore (162) without being in electrical communication with the inner sidewall of bore (162), while maintaining thermal communication with the inner sidewall of bore (162).

Flexible circuit (600) of this example further includes a lateral portion (618) of substrate layer (617) that projects laterally relative to the adjacent portion of substrate layer (617). Thermal contacts (613, 616) are positioned on adjacent portion of substrate layer (617) such that thermal contacts (613, 616) are lateral to lateral portion (618), with distal portions of traces (612, 615) extending along substrate layer (617) to reach thermal contacts (613, 616). Third trace (621) extends along lateral portion (618). As shown in FIG. 14A-15, lateral portion (618) is folded over to position lateral portion (618) under the adjacent region of substrate layer (617). The folded-over lateral portion (618) may act as a spring, similar to the partially compressed spacer layer (221) of flexible circuit (200), folded-over distal portion (318) of flexible circuit (300), and folded-over lateral portion (418) of flexible circuit (400), to resiliently bias thermocouple assembly (610) radially outwardly relative to the central longitudinal axis (L-L). This effect may in turn minimize the distance between thermal contacts (613, 616) and the outer surface of cylindraceous body (156), which may maximize the sensitivity of thermal contacts (613, 616) to the temperature of cylindraceous body (156) and tissue in contact with cylindraceous body (156). This may in turn maximize the sensitivity of junctions (625) to the temperature of cylindraceous body (156) and tissue in contact with cylindraceous body (156). Folded-over lateral portion (618) may thus promote thermal conductivity between thermocouple assembly (610) and cylindraceous body (156).

Further, folded-over lateral portion (618) may tend to provide securement of thermocouple assembly (610) within bore (162) by expanding the effective cross-sectional profile of thermocouple assembly (610). In some cases, the resilient bias of folded-over lateral portion (618) urges lateral portion (618) toward a flat/unfolded configuration, such that lateral portion (618) bears against the inner sidewall of bore (162), thereby enhancing friction to secure thermocouple assembly (610) within bore (162).

Positioning and securement of thermocouple assembly (610) within bore (162) may be further supplemented with an adhesive (627) to thus adhere thermocouple assembly (610) relative to distal tip member (142). In some cases where an adhesive is used, and while not shown in the present drawings, at least some of the adhesive may occupy a space formed between the folded-over lateral portion (618) and the underside of third trace (621) and substrate layer (617) (or the underside of an additional electrically insulating layer (not shown) that is positioned under third trace (621)). By way of example only, such adhesive may be provided via thermoplastic polyurethane elastomer that is introduced into bore (162) after thermocouple assembly (610) has been fully seated in bore (162). By way of further example only, such thermoplastic polyurethane elastomer may be doped with diamond powder and may be cured with ultraviolet light. Alternatively, any other suitable kind of adhesive may be used, and any other suitable process may be used to bond thermocouple assembly (610) relative to distal tip member (142).

### VII. Examples of Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

An apparatus comprising: (a) a shaft assembly having a distal end; (b) an end effector positioned at the distal end of the shaft assembly, the end effector including a tip member, the tip member being operable to apply electrical energy to tissue, the tip member defining a bore, the bore having an inner sidewall; and (c) a flexible circuit, the flexible circuit including a thermocouple positioned within the bore of the tip member, the thermocouple being configured to sense a temperature of the tip member, the flexible circuit further comprising a deformable feature, the deformable feature resiliently urging the thermocouple toward the inner sidewall of the bore.

### Example 2

The apparatus of Example 1, the flexible circuit further including: (i) a first trace extending to the thermocouple, (ii) a second trace extending to the thermocouple, and (iii) an insulating layer configured to electrically insulate the first trace and the second trace relative to the tip member.

### Example 3

The apparatus of any of Examples 1 through 2, further comprising an adhesive, the thermocouple being secured inside the bore of the tip member via the adhesive.

### Example 4

The apparatus of any of Examples 1 through 3, the deformable feature including a deformable spacer layer.

### Example 5

The apparatus of any of Examples 1 through 4, the flexible circuit further including a flexible substrate, the deformable feature comprising a distal portion of a flexible substrate, the distal portion being configured to extend distally relative to the thermocouple, the distal portion being bent proximally to thereby resiliently urge the thermocouple toward the inner sidewall of the bore.

### Example 6

The apparatus of any of Examples 1 through 5, the flexible circuit further including a flexible substrate, the deformable feature comprising a lateral portion of a flexible substrate, the lateral portion being configured to extend laterally relative to the thermocouple, the distal portion being bent inwardly to thereby resiliently urge the thermocouple toward the inner sidewall of the bore.

### Example 7

The apparatus of any of Examples 1 through 6, the flexible circuit further including: (i) a first conductive feature comprising a first type of electrically conductive material, (ii) a second conductive feature comprising a second type of electrically conductive material, the second type of electrically conductive material being different from the first type of electrically conductive material, (iii) an electrically insulative layer separating the first conductive feature from the second conductive feature, and (iv) a first junction electrically coupling the first conductive feature with the second conductive feature.

### Example 8

The apparatus of Example 7, the first type of electrically conductive material comprising copper, the second type of electrically conductive material comprising constantan.

### Example 9

The apparatus of any of Examples 7 through 8, the electrically insulative layer comprising polyimide.

### Example 10

The apparatus of any of Examples 7 through 9, the flexible circuit further including: (i) a third conductive feature comprising the first type of electrically conductive material, the electrically insulative layer separating the third conductive feature from the second conductive feature, and (ii) a second junction electrically coupling the third conductive feature with the second conductive feature.

### Example 11

The apparatus of any of Examples 1 through 10, the flexible circuit further including: (i) a distal portion, the thermocouple being positioned along the distal portion, and (ii) a proximal portion, the proximal portion including an arrangement of solder pads, the arrangement of solder pads being in electrical communication with the thermocouple.

### Example 12

The apparatus of Example 11, further comprising a printed circuit board including an arrangement of contact pads, each solder pad of the flexible circuit being in electrical communication with a respective contact pad of the arrangement of contact pads.

### Example 13

The apparatus of any of Examples 1 through 12, further comprising a handle, the flexible circuit extending from the handle, through the shaft assembly, and to the end effector.

### Example 14

The apparatus of any of Examples 1 through 13, the end effector further including an insulating layer positioned between the tip member and the thermocouple to thereby electrically insulate the thermocouple from the tip member.

### Example 15

The apparatus of any of Examples 1 through 14, the catheter further including a plurality of thermocouples, the plurality of thermocouples including the thermocouple of the flexible circuit, the plurality of thermocouples being angularly spaced apart from each other within the tip member.

### Example 16

An apparatus comprising: (a) a handle including a printed circuit board; (b) a shaft assembly extending distally relative to the handle, the shaft assembly having a distal end; (c) an end effector positioned at the distal end of the shaft assembly; and (d) a flexible circuit extending along the shaft assembly, the flexible circuit having a thermocouple positioned within the end effector, the thermocouple being configured to sense a temperature of the end effector, a distal portion of the flexible circuit being deformed within the end effector to thereby resiliently promote thermal conductivity between the thermocouple and the end effector, the flexible circuit being in electrical communication with the printed circuit board.

### Example 17

The apparatus of Example 16, the distal portion including one or more of a compressible spacer, a foldable distal projection, or a foldable lateral projection.

### Example 18

A method of attaching a thermocouple assembly to an end effector of a catheter, the thermocouple assembly being part of a flexible circuit, the flexible circuit including a deformable member, the deformable member being configured to transition from a non-deformed state to a deformed state, the deformable member being resiliently biased toward the non-deformed state, the method comprising: (a) deforming the deformable member of the flexible circuit to thereby provide the deformable member in the deformed state; (b) positioning the thermocouple and the deformable member in a bore of the end effector while the deformable member is in the deformed state, the deformable member resiliently urging the thermocouple toward a sidewall of the bore; and (c) securing the flexible circuit relative to the end effector while the thermocouple and the deformable member are in the bore of the end effector.

### Example 19

The method of Example 18, the deformable member comprising a spacer layer, the act of deforming the deformable member comprising compressing at least a portion of the spacer layer.

### Example 20

The method of any of Examples 18 through 19, the deformable member comprising a projecting portion of a flexible substrate, the projecting portion projecting distally or laterally relative to an adjacent region of the flexible substrate, the act of deforming the deformable member comprising folding the projecting portion.

### VII. Miscellaneous

Any of the instruments described herein may be cleaned and sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, hydrogen peroxide, peracetic acid, and vapor phase sterilization, either with or without a gas plasma, or steam.

It should be understood that any of the examples described herein may include various other features in addition to or in lieu of those described above. By way of example only, any of the examples described herein may also include one or more of the various features disclosed in any of the various references that are incorporated by reference herein.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Having shown and described various versions of the present invention, further adaptations of the methods and systems described herein may be accomplished by appropriate modifications by one skilled in the art without departing from the scope of the present invention. Several of such potential modifications have been mentioned, and others will be apparent to those skilled in the art. For instance, the examples, versions, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. An apparatus comprising:
(a) a shaft assembly having a distal end;
(b) an end effector positioned at the distal end of the shaft assembly, the end effector including a tip member, the tip member being operable to apply electrical energy to tissue, the tip member defining a bore, the bore having an inner sidewall; and
(c) a flexible circuit, the flexible circuit including a thermocouple positioned within the bore of the tip member, the thermocouple being configured to sense a temperature of the tip member, the flexible circuit further comprising a deformable feature, the deformable feature resiliently urging the thermocouple toward the inner sidewall of the bore.

2. The apparatus of claim 1, the flexible circuit further including:
(i) a first trace extending to the thermocouple,
(ii) a second trace extending to the thermocouple, and
(iii) an insulating layer configured to electrically insulate the first trace and the second trace relative to the tip member.

3. The apparatus of claim 1, further comprising an adhesive, the thermocouple being secured inside the bore of the tip member via the adhesive.

4. The apparatus of claim 1, the deformable feature including a deformable spacer layer.

5. The apparatus of claim 1, the flexible circuit further including a flexible substrate, the deformable feature comprising a distal portion of a flexible substrate, the distal portion being configured to extend distally relative to the thermocouple, the distal portion being bent proximally to thereby resiliently urge the thermocouple toward the inner sidewall of the bore, or the deformable feature comprising a lateral portion of a flexible substrate, the lateral portion being configured to extend laterally relative to the thermocouple, the distal portion being bent inwardly to thereby resiliently urge the thermocouple toward the inner sidewall of the bore.

6. The apparatus of claim 1, the flexible circuit further including:
(i) a first conductive feature comprising a first type of electrically conductive material,
(ii) a second conductive feature comprising a second type of electrically conductive material, the second type of electrically conductive material being different from the first type of electrically conductive material,
(iii) an electrically insulative layer separating the first conductive feature from the second conductive feature, and
(iv) a first junction electrically coupling the first conductive feature with the second conductive feature.

7. The apparatus of claim 6, the first type of electrically conductive material comprising copper, the second type of electrically conductive material comprising constantan and/or the electrically insulative layer comprising polyimide.

8. The apparatus of claim 7, the flexible circuit further including:
(i) a third conductive feature comprising the first type of electrically conductive material, the electrically insulative layer separating the third conductive feature from the second conductive feature, and
(ii) a second junction electrically coupling the third conductive feature with the second conductive feature.

9. The apparatus of claim 1, the flexible circuit further including:
(i) a distal portion, the thermocouple being positioned along the distal portion, and
(ii) a proximal portion, the proximal portion including an arrangement of solder pads, the arrangement of solder pads being in electrical communication with the thermocouple.

10. The apparatus of claim 9, further comprising a printed circuit board including an arrangement of contact pads, each solder pad of the flexible circuit being in electrical communication with a respective contact pad of the arrangement of contact pads and/or a handle, the flexible circuit extending from the handle, through the shaft assembly, and to the end effector.

11. The apparatus of claim 1, the end effector further including an insulating layer positioned between the tip member and the thermocouple to thereby electrically insulate the thermocouple from the tip member.

12. The apparatus of claim 1, the catheter further including a plurality of thermocouples, the plurality of thermocouples including the thermocouple of the flexible circuit, the plurality of thermocouples being angularly spaced apart from each other within the tip member.

13. An apparatus comprising:
(a) a handle including a printed circuit board;
(b) a shaft assembly extending distally relative to the handle, the shaft assembly having a distal end;
(c) an end effector positioned at the distal end of the shaft assembly; and
(d) a flexible circuit extending along the shaft assembly, the flexible circuit having a thermocouple positioned within the end effector, the thermocouple being configured to sense a temperature of the end effector, a distal portion of the flexible circuit being deformed within the end effector to thereby resiliently promote thermal conductivity between the thermocouple and the end effector, the flexible circuit being in electrical communication with the printed circuit board.

14. The apparatus of claim 13, the distal portion including one or more of a compressible spacer, a foldable distal projection, or a foldable lateral projection.

15. A method of attaching a thermocouple assembly to an end effector of a catheter, the thermocouple assembly being part of a flexible circuit, the flexible circuit including a deformable member, the deformable member being configured to transition from a non-deformed state to a deformed state, the deformable member being resiliently biased toward the non-deformed state, the method comprising:
(a) deforming the deformable member of the flexible circuit to thereby provide the deformable member in the deformed state;
(b) positioning the thermocouple and the deformable member in a bore of the end effector while the deformable member is in the deformed state, the deformable member resiliently urging the thermocouple toward a sidewall of the bore; and
(c) securing the flexible circuit relative to the end effector while the thermocouple and the deformable member are in the bore of the end effector.

16. The method of claim 15, the deformable member comprising i) a spacer layer, the act of deforming the deformable member comprising compressing at least a portion of the spacer layer, and/or ii) a projecting portion of a flexible substrate, the projecting portion projecting distally or laterally relative to an adjacent region of the flexible substrate, the act of deforming the deformable member comprising folding the projecting portion.
